# EUROPEAN PATENT APPLICATION

(11) **EP 3 685 823 A1**
(43) Date of publication of application: **29.07.2020**
(21) Application number: 19804607.0
(22) Date of filing: 21.01.2019
(51) Int. Cl.: A61K 8/81, A61Q 17/00

(54) **SPRAY FOR FORMING PROTECTIVE FILM ON SKIN SURFACE**

(30) Priority: 27.11.2018 JP 2018220968
(71) Applicant: EISHO CHEMICAL INDUSTRY Co., Ltd., Anjo-shi Aichi 4460057 (JP)
(72) Inventor: SUZUKI Hiroaki, Anjo-shi Aichi 4460057 (JP)
(74) Representative: Prüfer & Partner mbB Patentanwälte · Rechtsanwälte
(86) International application number: PCT/JP2019/001639
(87) International publication number: WO 2020/110325

(57) **Abstract**

A protective film forming spray for skin surface includes a spray bottle filled with an undiluted spray solution and a propellant, the undiluted spray solution containing 2 to 40 mass % of water-soluble resin and 2 to 40 mass % of water-insoluble resin so that a total content of the water-soluble resin and the water-insoluble resin is 15 to 50 mass % and a remainder is solvent.

## Description

### TECHNICAL FIELD

The present invention relates to a spray for easily forming a protective film on skin surface, the protective film including a protective film for preventing a person from getting a shoe sore and a protective film for preventing the hand(s) from becoming dirty.

### BACKGROUND ART

For instance, if a person wears new shoes or wears unfit shoes, he/she gets a blister(s) on her/his heel(s), toe(s), and other portions, leading to trouble walking. Therefore, a shoe sore prevention tape as disclosed in Patent Document 1, a shoe sore prevention pad as disclosed in Patent Document 2, and others have been developed and widely used.

### RELATED ART DOCUMENTS

### PATENT DOCUMENTS

Patent Document 1: JP-A-2018-27277
Patent Document 2: JP-A-2002-369836

### SUMMARY OF INVENTION

### PROBLEMS TO BE SOLVED BY THE INVENTION

However, the above shoe sore prevention tape disclosed in Patent document 1, shoe sore prevention pad disclosed in Patent Document 2, and others would cause problems that they need a troublesome using manner and they are hard to stick on a precise position. Such problems could not be easily addressed while a user is out.

There are further problems that the above products look bad or they cause positional displacement or peel-off during use, resulting in no protection effect. In particular, women who give great weight to fashionability could not obtain a feeling of satisfaction therefrom.

In contrast, an aerosol spray forms a transparent resin film coating by quick spraying on a skin to prevent shoe sores has been commercially available ("PreHeels" (Registered trademark): Sosu Company Limited). However, such a product may be problematic in strong solvent odor. Further, the transparent resin film coating may be less easily washed away. That is, the transparent resin film coating is hard to remove off even when it is washed with water or hot water. Thus, the transparent resin film coating has to be washed away with a soap, which is troublesome.

In other cases, for example, in working places where workers frequently touch oil or grease during work or in dusty working places with powder dust, such as a service garage, a gas/petrol station, a paint factory, and a farm, particles of dirt, grease/oil dirt, and others may stick between wrinkles of a hand or finger or between a nail and a skin. In those cases, even if the workers gently wash their hands after work, the dirt could not be perfectly washed out.

In view of the foregoing problems, therefore, there is a demand for development of a protective film forming spray for skin surface to easily form a protective film for preventing the hand(s) from becoming dirty.

The present invention has been made in view of the circumstances to solve the above problems and has a purpose to provide a protective film forming spray for skin surface, the spray being configured to easily form a protective film for preventing a user from getting a shoe sore and a protective film for preventing user's hands from becoming dirty, the protective film being resistant to a small volume of water and resistant to melting due to perspiration leading to a sticky state, the protective film being able to maintain a shoe sore prevention effect and a dirt prevention effect for prolonged time period term.

### MEANS OF SOLVING THE PROBLEMS

To achieve the above purpose, one aspect of the invention provides a protective film forming spray for skin surface, the spray comprising: a spray bottle; and an undiluted spray solution and a propellant filled in the spray bottle, the undiluted spray solution containing 2 to 40 mass% of water-soluble resin and 2 to 40 mass% of water-insoluble resin so that a total content of the water-soluble resin and the water-insoluble resin is 15 to 50 mass% and a remainder is solvent.

According to the above aspect, the water-soluble resin can make the protective film be easily washed away by water washing after usage. In contrast, the water-insoluble resin can prevent the protective film from becoming sticky due to perspiration or the like during use.

In the foregoing aspect, preferably, the protective film forming spray further contains one or more types among a lubricating agent, a perfume material, a moisturizer, a deodorant, an antibacterial agent, a surface active agent, a colorant in addition to the undiluted spray solution.

According to the above aspect, for example, when the lubricating agent is added, the lubricity is enhanced, thereby reducing shoe sores due to friction. This can also prevent a stocking from running. When the moisturizer is added, it can provide moisture to roughened and cracked heels due to drying or other portions.

When the perfume material and the colorant are added, they can add perfume and color to the skin. Further, when the deodorant and the antibacterial agent are added, they enable odor eliminating and antibacterial actions on the skin. When the surface active agent is added, it can produce effects to prevent dirt from sticking and further to easily remove dirt by hand wash. Those functions can be arbitrarily added according to usage purposes of the film.

In the foregoing aspect, preferably, a ratio of the undiluted spray solution to the propellant is 1:1 to 1:4.

According to the above aspect, a spraying state can be kept stable and good, so that the protective film can be formed uniformly.

### EFFECTS OF THE INVENTION

According to the protective film forming spray for skin surface according to the present invention, it is possible to easily form such a film by spraying as to be high in protective effect, durable for prolonged time period, and easy to remove after use.

### MODE FOR CARRYING OUT THE INVENTION

A detailed description of a preferred embodiment of the present invention will now be given below. In the present invention, an undiluted spray solution contains 2 to 40 mass% of a water-soluble resin and 2 to 40 mass% of a water-insoluble resin by mixing percentage in the total mass of the undiluted spray solution, and the total mixing percentage of the water-soluble resin and the water-insoluble resin is 15 to 50 mass%.

The water-soluble resin is added in order to facilitate dissolution of a protective film in a solvent and to enable removal of a protective film by water washing after use.

Concretely, the water-soluble resin may include for example polyvinylpyrrolidone (PVP), polyvinylpyrrolidone vinyl acetate copolymer (PVP/VA), acrylic resin alkanolamine.

The content of the water-soluble resin is preferably 2 to 40 mass% of the total mass of the undiluted spray solution. If the content of the water-soluble resin is less than 2 mass% of the total mass of the undiluted spray solution, the protective film is hard to be removed by water washing. To the contrary, if the content of the water-soluble resin exceeds 40 mass% of the total mass of the undiluted spray solution, this resin is hard to be completely dissolved in a solvent. More preferably, the content of the water-soluble resin falls within the range of 10 to 20 mass% of the total mass of the undiluted spray solution.

The water-insoluble resin is added in order to enhance wet resistance to prevent the occurrence of stickiness, thereby improving the feeling of use, and also to prevent the occurrence of stickiness of a protective film due to perspiration and others during use.

Concretely, the water-insoluble resin may include for example hydroxyethyl acrylate-methoxyethyl acrylate copolymer, vinylpyrrolidone vinyl acetate copolymer, acrylic acid alkyl ester - methacrylic acid alkyl ester -- diacetone acrylamide - methacrylic acid copolymer, vinyl acetate - crotonic acid copolymer, N-methacryloyloxyethyl N,N-dimethylammonium-α-N-methylcarboxybetaine- alkyl methacrylate copolymer.

The content of the water-insoluble resin is preferably 2 to 40 mass% of the total mass of the undiluted spray solution. If the content of the water-insoluble resin is less than 2 mass% of the total mass of the undiluted spray solution, it is difficult to enhance the wet resistance to prevent the occurrence of stickiness. To the contrary, if the content of the water-insoluble resin exceeds 40 mass% of the total mass of the undiluted spray solution, this resin is hard to be completely dissolved in a solvent. More preferably, the content of the water-insoluble resin falls within the range of 10 to 20 mass% of the total mass of the undiluted spray solution.

Furthermore, the total mixing percentage of the water-soluble resin and the water-insoluble resin in the total mass of the undiluted spray solution is preferably in the range of 15 to 50 mass%. If the total mixing percentage of the water-soluble resin and the water-insoluble resin is less than 15 mass% of the total mass of the undiluted spray solution, it is hard to obtain a protective film non-sticky and excellent feeling of use. To the contrary, if the the total mixing percentage of the water-soluble resin and the water-insoluble resin exceeds 50 mass% of the total mass of the undiluted spray solution, those resins are hard to be completely dissolved in a solvent. More preferably, the total mixing percentage of the water-soluble resin and the water-insoluble resin falls within the range of 25 to 40 mass%.

For the mixing percentages of the water-soluble resin and the water-insoluble resin, several series of tests were conducted and, as a result thereof, it was ascertained that when the water-soluble resin and the water-insoluble resin were mixed at a ratio of 1:1, the most excellent effect was obtained.

The remainder is a solvent, such as ethanol. The undiluted spray solution obtained as above may be additionally mixed with one or more types among a lubricating agent, a moisturizer, a deodorant, an antibacterial agent, a surface active agent, and a colorant, so that a higher-quality protective film is formed.

For example, when silicone is added as the lubricating agent, the protective film can exhibit an improved slip performance and can adequately prevent the occurrence of shoe sores.

The undiluted spray solution composed as above is mixed with a propellant to form an aerosol. Such an aerosol makes it possible to form a thin uniform film. This aerosol is also excellent in handling ease and storing ease.

The propellant may include for example liquefied gas, such as dimethyl ether (DME) and liquefied petroleum gas (LPG), and compressed gas, such as nitrogen, carbon dioxide, nitric oxide gas, and compressed air. However, if only the liquefied petroleum gas (LPG) is used or if the content ratio of the liquefied petroleum gas is high, the resin is not dissolved therein. Thus, this is not preferable.

The undiluted spray solution and the propellant to be filled are preferably mixed so that a mixing ratio of the undiluted spray solution to the propellant is 1:1 to 1:4 in mass ratio. If the ratio of the propellant to the undiluted spray solution is less than 1:1 (that is, if the mixing percentage of the propellant in the total mass of a mixture of the undiluted spray solution and the propellant is less than 50 mass%), the undiluted spray solution might not be sprayed out to the last drop. Further, it would take more time to dry the protective film, leading to low usability.

Meanwhile, if the mixing ratio of the undiluted spray solution to the propellant is more than 1:4 (that is, the mixing percentage of the propellant in the total mass of the mixture of the undiluted spray solution and the propellant is more than 80 mass%), the undiluted spray solution is too small in amount to instantaneously form a protective film having a sufficient thickness. More preferably, the mixing ratio of the undiluted spray solution to the propellant is in the range of 1:2 to 1:3.

### (Examples)

Examples of the present invention will be described below.

As shown in composition examples in Table 1, an undiluted spray solution was generated mainly containing: water-soluble resin: acrylic resin alkanolamine (GOO Chemical Co., Ltd., "Plascize L53D Color A"), water-insoluble resin: alkyl acrylate - alkyl methacrylate - diacetone acrylamide - methacrylic acid copolymer (GOO Chemical Co., Ltd., "Plascize L53"), and ethanol.

In addition, as a component for enhancing the slip performance, silicone (DOW CORNING TORAY, "SH556 FLUID" and DOW CORNING TORAY, "SH245 FLUID") was added. Further, as a plasticizer, polyhydric alcohol (1.3-BG) was added. The undiluted spray solution and the propellant (DME) were filled at a mixing ratio of 1:2 in mass ratio into a spray bottle. Thus, a spray for forming a protective film on skin surface was produced.

The produced protective film forming spray was sprayed onto a heel to form thereon a protective film for shoe sore prevention. After a lapse of 3 minutes from spraying, the protective film was dried into a transparent film fit with the skin. This protective film was inconspicuous in appearance.

The produced protective film was subjected to inspections about the feeling of use (stickiness), the film strength (durability), the effect of preventing a shoe sore, the washability (the ease of washing-away), and others. The results of those inspections are shown in in Table 1, which reveals that each produced protective film is evaluated as being good as a protective film for shoe sore prevention.

This evaluation was made by four stages; "⊚: very good", "○: good", "Δ: not good and not bad", and "×: bad".

In every example, the spray could evenly spray the solution and thus a thin uniform protective film could be formed on a heel. It is verified that this protective film is high in film strength, good in slip performance, and can prevent the occurrence of shoe sore for prolonged time period.

It is also verified that even if a user becomes sweaty or gets wet with water during use, the protective film can be maintained in a transparent thin-film state without causing stickiness of the skin. It is furthermore found that, after use, the protective film can be easily swept away by water washing and any film is not left on the foot.

The protective film shown in each example as above could exhibit an excellent effect as a shoe sore prevention film.

### (Comparative Example)

An undiluted spray solution made of the same components as those in the examples was prepared at each ratio indicated in the composition examples shown in Table 2. This undiluted spray solution and a propellant (DME) were filled at a mixing ratio of 1:2 in mass ratio into a spray bottle. Thus, a spray for forming a protective film on skin surface was produced.

The produced protective film forming spray was sprayed onto a heel to form thereon a protective film for shoe sore prevention. As in the examples, the produced protective film was subjected to inspections about the feeling of use (stickiness), the film strength (durability), the effect of preventing a shoe sore, the washability (the ease of washing-away), and others.

The results of those inspections are shown in Table 2, which reveals that every protective film caused a shoe sore during use and thus could not function as a shoe sore prevention protective film. If the content of either the water-soluble resin or the water-insoluble resin was too much or if the total amount of those two resins was too much, a mixture of the undiluted spray solution and the propellant was sprayed into a spider-web-like state. Thus, each protective film could not be formed as a uniform thin film on the heel.

If the content of the water-soluble resin was small, the washability (the ease of washing-away) was poor. If the content of the water-insoluble resin was small, the feeling of use was poor and the film became sticky if a user became sweaty or got wet with water during use. If the total content of the water-soluble resin and the water-insoluble resin was small, the film strength (the durability) was poor, resulting in a phenomenon that the film was easily torn.

**Table 1**

| Mixing component | Example 1 | Example 2 | Example 3 | Example 4 | Example 5 | Example 6 |
|---|---|---|---|---|---|---|
| 95° Ethanol | 57 | 57 | 60 | 60 | 60 | 55 |
| Plascize L-53D Color A | 33 | 3 | 16.5 | 11 | 22 | 33 |
| Plascize L-53 | 3 | 33 | 16.5 | 22 | 11 | 3 |
| 1.3-BG (butylene glycol) | | | | | | 2 |
| SH556 FLUID | 3 | 3 | 3 | 3 | 3 | 3 |
| SH245 | 4 | 4 | 4 | 4 | 4 | 4 |
| Evaluation Item | Example 1 | Example 2 | Example 3 | Example 4 | Example 5 | Example 6 |
| Film Strength | ○ | ○ | ⊚ | ⊚ | ○ | ○ |
| Feeling of use (Stickiness) | Δ | ⊚ | ⊚ | ○ | Δ | Δ |
| Cost | ⊚ | ○ | ⊚ | ○ | ⊚ | ○ |
| Washability (Ease of washing-away of resin) | ⊚ | Δ | ⊚ | ○ | ⊚ | ○ |
| Spraying state | ⊚ | ○ | ⊚ | ○ | ⊚ | ○ |
| Effect in actual use | ○ | ○ | ⊚ | ○ | ○ | ○ |

**Table 2**

| Mixing component | Comparative Example 1 | Comparative Example 2 | Comparative Example 3 | Comparative Example 4 | Comparative Example 5 | Comparative Example 6 |
|---|---|---|---|---|---|---|
| 95° Ethanol | 43 | 43 | 62 | 62 | 83 | 33 |
| Plascize L-53D Color A | 50 | 0 | 1 | 30 | 5 | 30 |
| Plascize L-53 | 0 | 50 | 30 | 1 | 5 | 30 |
| 1.3-BG (butylene glycol) | | | | | | |
| SH556 FLUID | 3 | 3 | 3 | 3 | 3 | 3 |
| SH245 | 4 | 4 | 4 | 4 | 4 | 4 |
| Evaluation Item | Comparative Example 1 | Comparative Example 2 | Comparative Example 3 | Comparative Example 4 | Comparative Example 5 | Comparative Example 6 |
| Film Strength | ○ | ⊚ | ⊚ | ○ | × | ⊚ |
| Feeling of use (Stickiness) | × | ○ | ○ | × | ○ | ○ |
| Cost | ○ | ○ | ○ | ⊚ | ⊚ | × |
| Washability (Ease of washing-away of resin) | ⊚ | × | × | ⊚ | ⊚ | Δ |
| Spraying state | × (sprayed into spider-web like state) | × (sprayed into spider-web like state) | ○ | ○ | ○ | × (sprayed into spider-web like state) |
| Effect in actual use | × | × | × | × | × | × |

As is clear from the above description, the present invention can easily form a protective film for preventing a shoe sore. This film is resistant to a small amount of water. Even if a user gets sweaty, the film coating is less likely to become melted into a sticky state and can maintain an effect of preventing a user from getting a shoe sore and an effect of preventing dirt from sticking for prolonged time period.

Moreover, for example, in a service garage, a gas/petrol station, a paint factory, a farm, and other places, dirt, grease/oil, and others may stick under the nails or penetrate the skin during work. In such a case, dirt, grease/oil, and others could not be removed even by washing.

However, a user only has to form a protective film on the skin or other portions with the spray in the present embodiment before work to prevent such dirt, grease/oil, and others from sticking under the nails or penetrating the skin. Since the protective film can be easily removed by washing after work, the protective film can exhibit an excellent effect for prevention of dirt.

The present invention is described above in the embodiment, but the present invention is not limited thereto and may be embodied in other specific forms without departing from the essential characteristics thereof.

Besides the shoe sore prevention film and the dirt prevention film exemplified in the embodiment, for example, the protective film forming spray are available for various skin surfaces.

### INDUSTRIAL APPLICABILITY

As is clear from the above description, the spray for forming a protective film on skin surface according to the present invention can easily form a film by spraying such that the film is high in protective effect, durable for prolonged time period, and easy to remove after use.

## Claims

1. A protective film forming spray for skin surface, the spray comprising: a spray bottle; and an undiluted spray solution and a propellant filled in the spray bottle, the undiluted spray solution containing 2 to 40 mass% of water-soluble resin and 2 to 40 mass% of water-insoluble resin so that a total content of the water-soluble resin and the water-insoluble resin is 15 to 50 mass% and a remainder is solvent.

2. The protective film forming spray for skin surface according to claim 1, further containing one or more types among a lubricating agent, a perfume material, a moisturizer, a deodorant, an antibacterial agent, a surface active agent, a colorant in addition to the undiluted spray solution.

3. The protective film forming spray for skin surface according to claim 1 or 2, wherein a ratio of the undiluted spray solution to the propellant is 1:1 to 1:4.
